# FASCICULE DE BREVET EUROPEEN

(11) **EP 1 978 356 B1**
(45) Date de publication et mention de la délivrance du brevet: **26.12.2018**
(21) Numéro de dépôt: 08300112.3
(22) Date de dépôt: 26.02.2008
(51) Int. Cl.: G01N 25/14, G01N 25/08, G01N 33/28, B01D 3/42, C10G 7/12

(54) **Procédé de gestion de l'analyse successive en ligne d'une série d'échantillons liquides portés à ébullition dans un ballon d'analyse**
Verfahren zur Steuerung der sukzessiven Online-Analyse einer Serie von flüssigen Proben, die in einem Analysekolben zum Sieden gebracht werden
Method for managing the successive in-line analysis of a series of liquid samples brought to boiling point in an analysis flask

(30) Priorité: 06.04.2007 FR 0754351
(43) Date de publication de la demande: 08.10.2008
(73) Titulaire: Instrumentation Scientifique de Laboratoire ISL, 14790 Verson (FR)
(72) Inventeur: Urvantsau, Viachaslau, 14120, MONDEVILLE (FR); Cleris, Hervé, 14220, CURCY SUR ORNE (FR)
(74) Mandataire: Livet, Marie-José

(56) Documents cités:
- EP-A- 0 406 093
- FR-A1- 2 410 818
- FR-A1- 2 789 490
- US-A- 3 849 260

## Description

La présente invention a pour objet un procédé de gestion de l'analyse successive en ligne d'une série d'échantillons liquides, en particulier d'échantillons de produits pétroliers dans un appareil d'analyse automatique équipé de moyens de commande et de régulation.

Lors de cette analyse, ces échantillons sont portés à ébullition dans un ballon d'analyse fermé comportant une partie sphérique coopérant avec un élément chauffant et se prolongeant par un col relié à des organes d'analyse.

Bien qu'il ne s'agisse aucunement là d'une caractéristique limitative de l'invention, un tel procédé de gestion est particulièrement adapté à la mesure des paramètres de distillation d'échantillons de produits pétroliers dans un appareil de distillation automatique en respectant une norme d'essai prédéfinie.

Il est connu que les paramètres de distillation des produits pétroliers sont représentatifs des performances de ces produits ainsi que des risques qu'ils peuvent faire encourir à leurs utilisateurs.

La détermination de ces paramètres présente notamment une grande importance dans le cas de carburants destinés à l'industrie automobile ou à l'aviation où les problèmes liés à la sécurité sont primordiaux.

Ces paramètres sont en particulier des tables ou des courbes représentant le pourcentage d'un échantillon évaporé selon la température pendant une distillation ou encore le volume du résidu et les pertes.

Les spécialistes peuvent déduire de ces paramètres quel sera le comportement d'un produit pétrolier donné dans une situation donnée et donc déterminer si ce produit peut ou non être utilisé en toute sécurité, ce de manière à obtenir les performances recherchées.

Il existe actuellement sur le marché différents appareils de distillation automatique permettant d'effectuer la mesure des paramètres de distillation d'un échantillon liquide en respectant une norme d'essai.

Ces appareils de distillation comportent en règle générale :
- un bâti fixe,
- un élément chauffant, notamment une résistance chauffante,
- au moins un ballon de distillation en verre dont le col peut être fermé par un bouchon d'obturation étanche muni d'un thermomètre et comporte une branche latérale destinée à être branchée sur un tube condenseur,
- une éprouvette de mesure permettant de recueillir le condensat et équipée d'organes de mesure de la quantité de condensat ainsi recueilli, et
- des moyens de commande et de régulation permettant de commander et de faire varier dans le temps une grandeur de fonctionnement de l'élément chauffant, notamment la température ou la puissance de cet élément de manière à obtenir des paramètres de distillation conformément à une norme d'essai.

Ces appareils de distillation automatique qui sont actuellement utilisés par tous les spécialistes pour caractériser les produits pétroliers liquides permettent d'obtenir des résultats fiables et reproductibles, donc représentatifs des échantillons analysés mais ne sont toutefois pas dénués d'inconvénients : ils sont en effet particulièrement lourds et encombrants ; de plus, le volume d'échantillon nécessaire pour effectuer une analyse est relativement important (de l'ordre de 100 ml) et la durée de celle-ci n'est pas inférieure à 45 mn.

Or, dans de nombreuses installations industrielles, il serait souhaitable de pouvoir effectuer, notamment à l'aide d'un appareil de ce type, une analyse en ligne d'une série d'échantillons liquides.

Cet appareil d'analyse pourrait à titre d'exemple être branché en dérivation sur une canalisation d'une installation d'élaboration industrielle de produits pétroliers.

Une telle analyse en ligne est toutefois difficilement envisageable compte tenu de la lenteur des réponses qui impliquent des temps d'attente particulièrement longs.

Dans ce contexte, il est à noter que l'on a récemment développé des appareils de laboratoire dits de « mini distillation » qui permettent de déterminer les paramètres de distillation d'échantillons liquides par des tests physiques ne durant chacun qu'environ 10 mn et ne nécessitant chacun qu'un volume très réduit d'échantillon (de l'ordre de 5 à 15 ml).

Ces appareils de « mini distillation » se distinguent en outre par le fait que les caractéristiques des échantillons analysés sont directement déterminées à partir de mesures de température et de pression, ce sans nécessiter de mesure du volume de condensat recueilli dans l'éprouvette de mesure.

De tels appareils de laboratoire, qui sont à titre d'exemple décrits dans le document FR 2 815 413, sont équipés de ballons d'analyse dont la partie sphérique coopère avec un élément chauffant tel qu'une résistance chauffante et dont le col comporte un capillaire latéral branché sur un tube condenseur et est fermé par un bouchon équipé d'un capteur de température qui plonge dans l'échantillon, ainsi que d'un capteur différentiel permettant de mesurer la pression régnant à proximité de l'entrée du capillaire.

Il pourrait être envisagé d'utiliser des appareils de « mini distillation » de ce type pour effectuer l'analyse successive en ligne d'une série d'échantillons liquides dans des installations industrielles de façon à remédier aux inconvénients susmentionnés des appareils d'analyse classiques.

Une transposition directe de ces appareils de laboratoire à la mise en oeuvre d'analyses en ligne à l'échelle industrielle est toutefois impossible pour plusieurs raisons liées en particulier à l'introduction successive des échantillons à analyser dans le ballon d'analyse et à la nécessité de rincer ce ballon après chaque test.

En effet, dans les appareils de « mini distillation », l'échantillon à analyser est en règle générale introduit au moyen d'une seringue, par le col du ballon d'analyse ce qui ne pose pas de problème dans le cas d'analyses séquentielles au laboratoire mais est impossible dans le cas d'appareils d'analyse en ligne branchés en dérivation sur des canalisations d'installations d'élaboration de produits industriels travaillant en continu dans lequel le ballon d'analyse doit être fixé au bâti de l'appareil.

Il est en outre difficilement envisageable d'équiper le col ou les parois latérales du ballon d'analyse d'éléments mécaniques supplémentaires tels que des tubes et des soupapes de nature à permettre d'introduire les échantillons à analyser à la partie supérieure de ce ballon vu que la présence de ces éléments supplémentaires nuirait à l'exactitude des mesures et diminuerait par suite la fiabilité de l'analyse, en particulier en raison de risques d'obstruction de ces éléments.

De manière similaire, il est impossible d'extraire le ballon d'analyse de l'appareil après chaque test pour le déboucher et le remplir d'un solvant approprié avant d'évacuer ce solvant par le col du ballon afin d'effectuer le rinçage nécessaire.

Il est par ailleurs à noter que lors de l'analyse d'échantillons liquides portés à ébullition, il est essentiel de stabiliser ce processus pour permettre d'obtenir des résultats fiables et reproductibles et en outre d'éviter que le système se charge en calamine.

A cet effet les spécialistes ont pris l'habitude d'introduire dans le ballon d'analyse, en plus de l'échantillon, un solide poreux ayant une surface développée importante telle que de la pierre ponce.

Une telle introduction est bien entendu exclue dans des appareils destinés à permettre d'analyser successivement en ligne et de manière automatique une série d'échantillons, notamment dans des installations d'élaboration industrielle de produits pétroliers.

Pour amorcer et stabiliser le processus d'ébullition, il a également déjà été proposé d'insuffler un gaz tel que de l'air ou de l'azote dans l'échantillon liquide introduit dans le ballon d'analyse.

Toutefois, une telle insufflation oblige à adjoindre des tubes ou éléments mécaniques supplémentaires tels que des soupapes au niveau du col ou des parois latérales du ballon d'analyse, ce qui est là encore de nature à nuire à l'exactitude et à la fiabilité des résultats obtenus.

Le document FR 2 789 490 décrit un appareil d'analyse équipé d'un bouilleur coopérant avec un élément chauffant, et des tubulures de liaison situées à la base dudit bouilleur - une première tubulure est utilisée pour injecter un flux gazeux pour homogénéiser la température d'ébullition, et la deuxième tubulure est utilisée pour le vidange.

La présente invention, telle que définie dans la revendication 1, a pour objet de proposer un procédé de gestion de l'analyse successive en ligne et en continu dans un appareil d'analyse automatique d'une série d'échantillons liquides, en particulier d'échantillons de produits pétroliers portés à ébullition dans un ballon d'analyse fermé de faible volume, en règle générale de l'ordre de 10 ml, de nature à permettre de remédier aux inconvénients susmentionnés.

Selon l'invention, un tel procédé de gestion est caractérisé par la succession des étapes suivantes :
- on introduit un premier échantillon dans le ballon d'analyse par une tubulure de liaison située à la base de la partie sphérique de ce ballon,
- on commande l'élément chauffant pour porter cet échantillon à ébullition et effectuer son analyse automatique en injectant en continu un faible flux gazeux, notamment un flux d'air ou d'azote dans la tubulure de liaison de façon à créer au sein de l'échantillon en cours d'analyse des petites bulles de nature à stabiliser son ébullition,
- après l'achèvement de l'analyse, on introduit un second échantillon faisant office de solvant dans le ballon d'analyse par la tubulure de liaison de façon à permettre le rinçage du résidu,
- on évacue le second échantillon du ballon d'analyse par la tubulure de liaison, par pressurisation de ce ballon et gravité, et
- on introduit un troisième échantillon dans le ballon d'analyse par la tubulure de liaison pour effectuer son analyse automatique et ainsi de suite.

Il est à noter que dans le cadre de cet exposé, les termes haut, bas, inférieur, supérieur... doivent être compris en se référant à un ballon d'analyse fixé au bâti de l'appareil dans une position de travail essentiellement verticale.

La mise en oeuvre du procédé de gestion conforme à l'invention est ainsi basée sur l'adjonction au ballon d'analyse, au niveau du point bas de la partie sphérique de ce ballon, d'une tubulure de liaison ayant en règle générale un diamètre de l'ordre du millimètre.

Selon l'invention, cette tubulure de liaison a trois fonctions distinctes.

La première de ces fonctions consiste à permettre l'introduction d'un échantillon à analyser dans le ballon d'analyse.

La seconde fonction consiste à permettre d'insuffler un flux gazeux dans cet échantillon pour amorcer et stabiliser son ébullition ; ce flux gazeux exerce parallèlement une poussée qui empêche tout retour de l'échantillon en cours d'analyse par la tubulure de liaison.

La troisième fonction consiste à permettre de vider le ballon d'analyse après rinçage en évacuant le solvant utilisé à cet effet ; il est à noter que le positionnement de la tubulure de liaison à la base de la partie sphérique du ballon d'analyse, c'est-à-dire au point bas de ce ballon, à proximité de l'élément chauffant est de nature à créer des conditions optimum pour permettre une telle évacuation.

Cette dernière est par ailleurs avantageusement facilitée par la pressurisation du ballon d'analyse, ce grâce à une ligne de pression équipant l'appareil.

Une autre caractéristique essentielle du procédé de gestion conforme à l'invention est liée à l'utilisation de l'échantillon en tant que solvant lors de l'étape de rinçage qui doit obligatoirement être mise en oeuvre après chaque test.

L'échantillon constitue donc ainsi son propre solvant.

Il est en outre à noter que pour donner satisfaction, un appareil permettant l'analyse successive, en ligne et en continu, d'une série d'échantillons - notamment dans des installations d'élaboration industrielle de produits pétroliers - doit pouvoir être utilisé pendant plusieurs semaines, sans maintenance.

Dans ce but, il est par suite impératif de maîtriser les problèmes de calamine.

A cet effet et selon une caractéristique préférentielle de l'invention, après une série d'analyses d'échantillons, on met en oeuvre une étape de régénération par thermodécomposition selon laquelle on chauffe le ballon d'analyse jusqu'à une température de l'ordre de 500°C en présence d'oxygène de façon à éliminer la calamine déposée et on rince les cendres et poudres ainsi formées en introduisant un autre échantillon faisant office de solvant dans le ballon d'analyse par la tubulure de liaison puis en l'évacuant par cette même tubulure par pressurisation et gravité.

Pendant cette étape de régénération, on peut insuffler un faible flux d'air ou d'oxygène par la tubulure de liaison de façon à disposer de l'oxygène nécessaire à la thermodécomposition de la calamine.

La fréquence de cette étape de régénération est bien entendu fonction du taux de dépôt de calamine par l'échantillon à analyser.

Selon une autre caractéristique particulièrement avantageuse de l'invention, on utilise un ballon d'analyse dont la partie sphérique est équipée, au niveau de sa base, d'une série de cavités de section essentiellement conique.

Ces cavités qui sont situées à proximité de l'élément chauffant permettent de créer des points de surchauffe qui entraînent la formation de petites bulles contribuant à la stabilisation de l'ébullition d'un échantillon en cours d'analyse.

L'action de ces cavités se superpose ainsi à celle du flux gazeux insufflé dans l'échantillon en cours d'analyse par la tubulure de liaison pour amorcer et stabiliser l'ébullition.

Le mode de réalisation de ces cavités peut être quelconque sans pour cela sortir du cadre de l'invention, à la condition qu'elles aient une section essentiellement conique.

Celles-ci peuvent à titre d'exemple être réalisées sous la forme d'évidements ponctuels uniformément répartis au niveau de la base de la partie sphérique du ballon d'analyse ou encore de petites rainures concentriques disposées autour de la tubulure de liaison.

Leur profondeur est bien entendu fonction de l'épaisseur de la paroi du ballon d'analyse au niveau de la base de la partie sphérique de ce ballon.

Selon l'invention, la présence de ces cavités au niveau de l'élément chauffant crée un gradient de température dans la profondeur des cavités : en effet, la température au point inférieur d'une cavité est toujours supérieure à la température moyenne de l'échantillon en cours d'ébullition.

Ce gradient de température entraîne une surchauffe ponctuelle de l'échantillon en cours d'ébullition à la partie interne de la cavité, de sorte que dans cette zone, l'ébullition commence plus tôt que dans la partie restante de l'échantillon.

Il en résulte que la partie interne des cavités correspond à la zone d'amorçage du processus d'ébullition dans laquelle on observe la formation de petites bulles qui contribuent à stabiliser ce processus.

Il est à noter que l'étape de régénération périodique permet également d'éliminer par thermodécomposition la calamine pouvant s'être déposée dans les cavités.

Celles-ci ne peuvent ainsi pas être le siège de phénomènes d'obstruction de nature à nuire à leur efficacité.

Selon l'invention, on utilise de préférence un ballon d'analyse métallique, en particulier en acier inoxydable sur la paroi externe duquel l'élément chauffant, notamment la résistance chauffante, peut avantageusement être soudé, au niveau de la base de la partie sphérique de ce ballon.

Il est en effet à noter que dans un processus industriel, l'utilisation d'un ballon métallique est toujours préférable à celle d'un ballon classique en verre.

Les caractéristiques du procédé de gestion qui fait l'objet de l'invention seront décrites plus en détail en se référant aux dessins non limitatifs annexés dans lesquels :
- la figure 1 est une vue schématique d'un ballon d'analyse utilisé lors de la mise en oeuvre du procédé de gestion conforme à l'invention,
- la figure 2 est un détail de la figure 1 au niveau de la paroi de ce ballon.

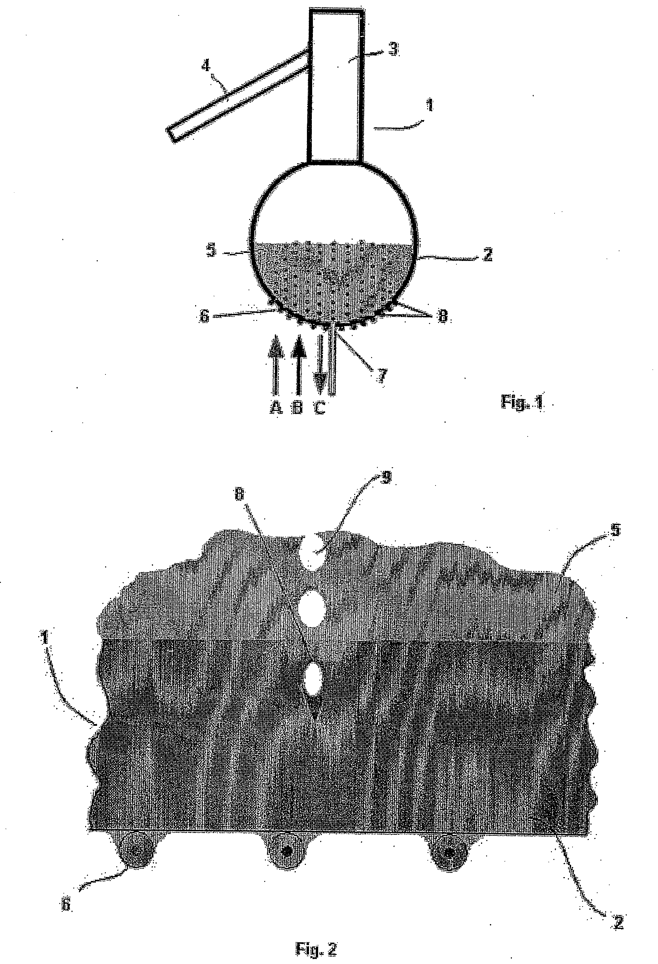

Selon la figure 1, le ballon d'analyse 1 est réalisé en acier inoxydable et comporte une partie sphérique 2 se prolongeant par un col 3 équipé d'un capillaire latéral 4.

Un échantillon 5 porté à ébullition est introduit dans le ballon d'analyse 1.

Selon les figures 1 et 2, une résistance chauffante 6 est soudée sur la paroi externe du ballon d'analyse 1, au niveau de la base de la partie sphérique 2 de ce ballon.

Selon la figure 1, le ballon 1 est équipé d'une tubulure de liaison 7 de faible diamètre montée à la base de la partie sphérique 2 de ce ballon.

Cette tubulure de liaison 7 a une triple fonction consistant à permettre d'introduire un échantillon à analyser dans le ballon 1 selon la flèche A, d'insuffler en continu un faible flux gazeux dans l'échantillon pendant l'analyse selon la flèche B, et d'évacuer le résidu par rinçage selon la flèche C après l'analyse.

Selon les figures 1 et 2, la partie sphérique 2 du ballon d'analyse 1 est équipée au niveau de sa base d'une série de cavités 8 ayant une section conique.

Comme représenté sur la figure 2, la présence de ces cavités au niveau de l'élément chauffant 6 crée un gradient de température dans la profondeur des cavités du ballon d'analyse 1, ce qui entraîne une surchauffe locale de l'échantillon à analyser dans cette zone, et par suite la formation de bulles 9 contribuant à stabiliser l'ébullition de cet échantillon.

## Revendications

1. Procédé de gestion de l'analyse successive en ligne et en continu d'une série d'échantillons liquides d'un produit dans un appareil d'analyse automatique branché en dérivation sur une canalisation d'une installation d'élaboration industrielle de ce produit et équipé d'un ballon d'analyse ayant un volume de l'ordre de 10 ml et comportant une partie sphérique coopérant avec un élément chauffant, comportant la succession des étapes suivantes :
- on introduit un premier échantillon du produit à analyser dans ce ballon d'analyse (1) par une tubulure de liaison (7) située à la base de la partie sphérique (2) du ballon,
- on commande l'élément chauffant (6) pour porter cet échantillon à ébullition et effectuer son analyse automatique en injectant en continu un faible flux gazeux, notamment un flux d'air ou d'azote dans la tubulure de liaison (7) de façon à créer au sein de l'échantillon en cours d'analyse des petites bulles de nature à stabiliser son ébullition,
- après l'achèvement de l'analyse, on introduit un second échantillon du produit à analyser faisant office de solvant dans le ballon d'analyse (1) par la tubulure de liaison (7) de façon à permettre le rinçage du résidu,
- on évacue le second échantillon du ballon d'analyse (1) par la tubulure de liaison (7), par pressurisation de ce ballon et gravité, et
- on introduit un troisième échantillon du produit à analyser dans le ballon d'analyse (1) par la tubulure de liaison (7) pour effectuer son analyse automatique et ainsi de suite.

2. Procédé selon la revendication 1,
**caractérisé en ce qu'**
après une série d'analyses d'échantillons, on met en oeuvre une étape de régénération par thermodécomposition selon laquelle on chauffe le ballon d'analyse (1) jusqu'à une température de l'ordre de 500°C en présence d'oxygène de façon à éliminer la calamine déposée et on rince les cendres et poudres ainsi formées en introduisant un autre échantillon du produit à analyser faisant office de solvant dans le ballon d'analyse par la tubulure de liaison (7), puis en l'évacuant par cette même tubulure (7) par pressurisation et gravité.

3. Procédé selon l'une quelconque des revendications 1 et 2,
**caractérisé en ce que**
l'on utilise un ballon d'analyse (1) dont la partie sphérique (2) est équipée, au niveau de sa base, d'une série de cavités (8) de section essentiellement conique permettant de créer des points de surchauffe entraînant la formation de petites bulles (9) contribuant à la stabilisation de l'ébullition d'un échantillon en cours d'analyse.

4. Procédé selon l'une quelconque des revendications 1 à 3,
**caractérisé en ce que**
l'on utilise un ballon d'analyse (1) métallique, en particulier en acier inoxydable.

5. Procédé selon la revendication 4,
**caractérisé en ce que**
l'élément chauffant (6) est soudé sur la paroi externe du ballon d'analyse (1), au niveau de la base de la partie sphérique (2) de ce ballon (1).

## Patentansprüche

1. Verfahren zur Leitung der sukzessiven und kontinuierlichen Online-Analyse einer Serie von flüssigen Proben eines Produkts in einer automatischen Analysenvorrichtung, die an eine Nebenkanalisation einer Einrichtung zur industriellen Ausarbeitung dieses Produkts angeschlossen und mit einem Analysekolben ausgestattet ist, der ein Volumen von etwa 10 ml aufweist und einen kugelförmigen Teil, der mit einem Heizelement zusammenwirkt, umfasst, umfassend die Aufeinanderfolge der folgenden Schritte:
- eine erste Probe des zu analysierenden Produkts wird durch eine Verbindungsleitung (7), die an der Basis des kugelförmigen Teils (2) des Kolbens angeordnet ist, in den Analysekolben (1) eingeführt,
- das Heizelement (6) wird gesteuert, um die Probe zum Sieden zu bringen und ihre automatische Analyse durchzuführen, indem ein geringer Gasstrom, insbesondere ein Luft- oder Stickstoffstrom in die Verbindungsleitung (7) kontinuierlich injiziert wird um innerhalb der Probe, die analysiert wird, kleine Blasen zu bilden, die naturgemäß ihr Sieden stabilisieren können,
- nach der Beendigung der Analyse wird eine zweite Probe des zu analysierenden Produkts, die als Solvent fungiert, durch die Verbindungsleitung (7) in den Analysekolben (1) eingeführt, um die Spülung des Rückstandes zu ermöglichen,
- die zweite Probe wird durch die Verbindungsleitung (7), durch Druckbeaufschlagung des Kolbens und durch Schwerkraft, aus dem Analysekolben (1) abgeführt, und
- eine dritte Probe des zu analysierenden Produkts wird durch die Verbindungsleitung (7) in den Analysekolben (1) eingeführt, um ihre automatische Analyse durchzuführen, und so weiter.

2. Verfahren gemäß Anspruch 1,
**dadurch gekennzeichnet, dass**
nach einer Serie von Probenanalysen ein Regenerationsschritt durch Thermozersetzung durchgeführt wird, nach dem der Analysekolben (1) bis zu einer Temperatur von etwa 500 °C in Gegenwart von Sauerstoff erhitzt wird, um den abgesetzten Galmei zu entfernen, und die so gebildeten Aschen und Pulver ausgespült werden, indem eine weitere Probe des zu analysierenden Produkts, die als Solvent fungiert, durch die Verbindungsleitung (7) in den Analysekolben eingeführt und dann durch dieselbe Leitung (7) durch Druckbeaufschlagung und Schwerkraft abgeführt wird.

3. Verfahren gemäß einem der Ansprüche 1 oder 2,
**dadurch gekennzeichnet, dass**
ein Analysekolben (1) verwendet wird, dessen kugelförmiger Teil (2) an seiner Basis mit einer Serie von Hohlräumen (8) mit einem im Wesentlichen konusförmigen Schnitt ausgestattet ist, die es ermöglichen, Überhitzungspunkte zu bilden, die die Bildung von kleinen Blasen (9) herbeiführen, die zur Stabilisierung des Siedens einer Probe, die analysiert wird, beitragen.

4. Verfahren gemäß einem der Ansprüche 1 bis 3,
**dadurch gekennzeichnet, dass**
ein metallischer Analysekolben (1) verwendet wird, insbesondere aus Edelstahl.

5. Verfahren gemäß Anspruch 4,
**dadurch gekennzeichnet, dass**
das Heizelement (6) an der Basis des kugelförmigen Teils (2) des Kolbens (1) auf die Außenwandung des Analysekolbens (1) aufgeschweißt wird.

## Claims

1. Method for managing the consecutive continuous in-line analysis of a series of liquid samples of a product in an automatic analysis device that is connected as a bypass to a pipe of an industrial development plant of said product and provided with an analysis flask having a volume of approximately 10 ml and comprising a spherical portion that interacts with a heating element,
comprising the following series of steps:
- introducing a first sample of the product to be analysed into said analysis flask (1) via a connection tube (7) located at the base of the spherical portion (2) of the flask,
- controlling the heating element (6) so as to bring said sample to the boil and carry out automatic analysis thereof by continuously feeding a weak flow of gas, in particular a flow of air or nitrogen, into the connection tube (7) so as to create small bubbles within the sample being analysed which stabilise the boiling,
- when the analysis is complete, introducing a second sample of the product to be analysed that acts as a solvent into the analysis flask (1) via the connection tube (7) so as to allow the residue to be rinsed,
- discharging the second sample from the analysis flask (1) via the connection tube (7) by means of pressurisation of said flask and gravity, and
- introducing a third sample of the product to be analysed into the analysis flask (1) via the connection tube (7) for carrying out automatic analysis of said sample, and so on.

2. Method according to claim 1, **characterised in that** after a series of sample analyses, a step of regeneration by means of thermal decomposition is implemented, according to which the analysis flask (1) is heated to a temperature of approximately 500°C in the presence of oxygen so as to remove the deposited scale, and the dust and powder formed as a result are rinsed by introducing another sample of the product to be analysed that acts as a solvent into the analysis flask via the connection tube (7), then discharging said sample via the same tube (7) by means of pressurisation and gravity.

3. Method according to either claim 1 or claim 2, **characterised in that** an analysis flask (1) is used of which the spherical portion (2) is provided, at the base thereof, with a series of cavities (8) having a substantially conical cross section that allow superheat points to be created which cause the formation of small bubbles (9) that contribute to the stabilisation of the boiling of a sample being analysed.

4. Method according to any of claims 1 to 3, **characterised in that** a metal analysis flask (1) is used, in particular one made of stainless steel.

5. Method according to claim 4, **characterised in that** the heating element (6) is soldered onto the outer wall of the analysis flask (1) at the base of the spherical portion (2) of said flask (1).
